# EUROPEAN PATENT APPLICATION

(11) **EP 4 737 974 A1**
(43) Date of publication of application: **06.05.2026**
(21) Application number: 25170204.9
(22) Date of filing: 11.04.2025
(51) Int. Cl.: G02B 21/08, G02B 23/24, G01N 15/0227, G01N 21/47, C12M 1/34, G01N 21/53, G01N 21/85

(54) **DEVICE FOR DIRECTING ILLUMINATION, INSTRUMENTS COMPRISING SAID DEVICE, AND METHODS OF PERFORMING CYTOMETRY**

(30) Priority: 04.11.2024 EP 24210649; 14.03.2025 EP 25163723
(71) Applicant: Mettler-Toledo GmbH, 8606 Greifensee (CH)
(72) Inventor: Link, Sandro, 8308 Illnau (CH); Gati, Rudolf, 5507 Mellingen (CH); Sobanski, Nicolas, 8606 Nänikon (CH)
(74) Representative: Mettler-Toledo

(57) **Abstract**

A device for directing illumination for a sample volume in microscopy, in particular in in-situ cytometry. The device comprises a body (611), the body having a distal front end (62) and an optical aperture provided (63) in the front end, and a reflector (621). The reflector is arranged distal from the front end and at a non-zero distance from the optical aperture and is configured to receive light emitted through the front end and to reflect at least part of the light received from the front end back into the optical aperture. The reflector is connected to the body by at least one cantilevering strut. The suggested device may be part of an objective lens unit (6).

## Description

### Technical Field

The herein claimed subject matter relates generally to instruments and methods applicable for in-situ cytometry. More in particular, it relates to the subject matter set forth in the claims.

### Background Art

Cytometry is applied to characterize biological cells, alive or death. Preferably, also for cytometry using the device according to the invention, the cells are in a liquid. For instance, density, number density, size and morphology of biological cells may be determined by microscopy. In the absence of reliable and quantitative in-line and in-situ measurement methods, bioengineers have to take samples to perform cytometry with off-line measurement devices. This requires taking a sample volume of content from the bioreactor within certain intervals, which increases the risk of contamination. In addition, it is time-consuming, and due to the sparse interval little statistics and no live information about cytometry is available to the operator. An in-situ applicable in-line cytometer would drastically reduce the complexity compared to off-line methods and would allow the process controller to monitor the cell count and cell condition close to real-time.

In-line imaging cytometry requires high-quality images of the biological cells. A key requirement for images with high quality is appropriate illumination of the objects to be observed. Since the cells within the bioreactor are typically moving fast due to the stirring of the bioreactor, the use of short light pulses of a few microseconds is preferred to avoid smearing and blurring of the image. This strongly limits the light energy collected per frame taken. In addition, the biological cells typically have a refractive index very close to the surrounding medium, further reducing the interaction with the light and therefore the object contrast. For biological cells in a liquid suspension, it was observed that back-scattered light can be several orders of magnitude smaller than light scattered in forward direction. Therefore, transmission microscopy, i.e., illuminating the objects to be observed from beyond the object when seen from the microscope objective, or, in still other words, with an illumination source emitting the light in a proximal direction and directing the light through the sample volume and towards the aperture of the microscope, results in drastically enhanced image quality compared to purely back-scattered light with the objects illuminated by distally travelling light. However, placing a light source underneath the objects respectively the sample volume for an in-situ cytometer within a bioprocess may yield certain drawbacks.

In-line or in-situ measurements are - in the case of a process, in particular a bioprocess- measurements which take place directly in the reactor respectively in the bioreactor or in a tube transporting the fluid to be monitored. In particular, the fluid to be monitored can be the liquid of the bioprocess comprising the medium and the biological cells.

US 6,809,862 suggests a microscope including a microscope arrangement having a specimen zone between a slide glass body and a lens cover glass, the lens cover glass closing the microscope in a distal direction. An illumination arrangement is provided distal from the sample zone and covered by the slide glass body and illuminates the sample zone through the sample zone with the illumination directed towards the microscope lens. Thus, the sample is viewed in transmitted light. A bulky unit comprising a light source, a power supply for the light source, and a collimator optics is arranged in front of, or distal from, the microscope and inside the reactor and influences flow patterns inside the reactor when applied for in-situ microscopy.

DE 40 32 002 teaches to apply transmission microscopy inside a bioreactor. For that purpose, a microscope is arranged outside the bioreactor and is separated from the interior of the bioreactor by a window. A bent tube containing an optical fiber is inserted into the bioreactor and is directed to emit light inside the bioreactor through a sample volume and towards the window, or the microscope, respectively. The insertion of the bent tube requires an additional dedicated port of the bioreactor.

US 4,515,445 discloses an optical system for transmitted-light microscopy with incident illumination. The therein suggested device comprises a common upright reflected light microscope provided on one side of an object and a retroreflection device which contains an optical system which images the object unreversed and upright on itself on the other side of the object. The object is placed on a microscope slide of the upright microscope. The purpose of the taught subject matter is to observe light which passes an object twice. US 4,515,445 gives no hint for application in in-situ cytometry, and the retroreflection device which needs to be placed distal from the object, or a sample volume, to be observed is rather bulky and thus unsuitable for in-situ cytometry.

GB 784,822 teaches to emit plane polarized light having a first plane of polarization to illuminate a sample and observe light witch is polarized in a plane of polarization perpendicular to the first plane of polarization. The sample is placed on a microscope slide. A quarterwave plate is rotatably arranged proximal from the microscope slide, i.e., in the optical path between the light source and the microscope slide, or the sample, respectively. A depolarizing reflector is arranged distal from the microscope slide. GB 784,822 essentially discloses an upright microscope. No hint or suggestion how to use the therein described teaching for in-situ cytometry is given.

US 2003/0011881 describes a confocal microscope applying a Nipkow-disk to scan a specimen. A deflection means is arranged distal from the specimen to be scanned to enable illumination of the specimen also in a direction towards the objective. Scanning the specimen implies, naturally, that the specimen needs to stay in place at least during one scan process. This makes the teaching unapplicable for in-line and in-situ cytometry.

There is a demand for an instrument which enables in-situ cytometry using in-situ transmission microscopy.

### Summary of invention

The herein claimed invention is defined by the appended claims.

It is an object of the present disclosure to suggest the subject matter initially mentioned. In one aspect, the herein disclosed subject matter shall support providing transmission illumination of objects in in-situ microscopy, in particular of objects being suspended in a sample liquid, for example a liquid medium of a bioprocess. In another aspect, the herein disclosed subject matter shall overcome at least some of the drawbacks of the art.

This is achieved by the subject matter set forth in the appended claims. Further effects and advantages of the disclosed subject matter, whether explicitly mentioned or not, will become apparent in view of the disclosure provided below. In a more particular point of view, the herein disclosed subject matter shall enable transmission microscopy inside a liquid.

Accordingly, disclosed is a device for directing illumination, in particular for a sample volume in microscopy, in particular in in-situ cytometry. In the case of cytometry, the objects to be observed are biological cells. The device comprises a body having a distal front end and an optical aperture provided in the front end. Further, the device comprises a reflector which is connected to the body and arranged distal from the front end and a non-zero distance from the optical aperture. The reflector is arranged and configured to receive light emitted through the front end of the body and to reflect at least part of the light received from the front end back into the optical aperture. This allows a particular robust and compact construction and, as explained in more details below, the scattering properties of many cells suspended in a liquid are such that it can be tolerated to observe the forward and backward image simultaneously.

Preferably, the light received by the reflector is emitted through the optical aperture. It is however also possible, to illuminate the reflector from a direction tilted with respect to the optical axis defined by the optical aperture and the reflector. For example, the reflector could be a curved mirror illuminated with ring light source surrounding the optical aperture and being tilted such that the reflected light appears collimated. In some embodiments, the aperture through which the emitted light leaves the body is the same as the optical aperture. In some embodiments, the front end comprises one or more additional apertures through which the emitted light can leave the body. Said additional apertures can for example be provided by being light guides embedded in the material of the body.

In embodiments, the device is such that the sample liquid comprising the objects to be observed can continuously flow into and out of the space between the reflector and the optical aperture. The reflector requires only a small space and at it is being connected to the body of the device, there is no need for additional support structures such as the ones commonly used by table-top microscopes designed for observing objects on slides.

A sample volume in the space between the optical aperture and the reflector may be observed through the optical aperture. Illumination for the sample volume may in instances be provided through the optical aperture. Illumination light emitted through the optical aperture is reflected by the reflector and travels back towards the optical aperture, thereby passing the sample volume. The illumination light is scattered by objects, such as particles or cells, in the sample volume, and the resulting forward scattered light travels through the optical aperture towards an observer or receiver. In instances, the intensity of forwards scattered light may be orders of magnitude larger than the intensity of backscattered light. The signal received from the objects thus is significantly greater than the signal which would be received if only the backscattered signal from illumination light travelling directly from the optical aperture and through the sample volume was observed.

In particular in the case when the light which is originally emitted from the optical aperture travels back from the reflector to the optical aperture along the same way as the light emitted from the optical aperture to the reflector, it will be appreciated that the light travels through the sample volume twice, and backscattered light as well as forward scattered light from the sample volume is observed through the optical aperture.

Depending on the distance of the object plane, which is observed, from the optical aperture, in instances also light which is forward scattered as the illumination light travels from the optical aperture to the reflector and light which is backscattered as the illumination light travels from the reflector towards the optical aperture may be observed. Said distance of the object plane from the optical aperture may for instance be set by adjusting the focal length of an objective or objective lens unit of a microscope having a field of view through the optical aperture.

If, for instance, an immersion lens, and more in particular a solid immersion lens, so-called SIL, is used to observe the sample volume, a relatively narrow depth of field, typically measuring only some µm along an axial or proximal-distal direction, or direction of view. The field of view captured by a suitable sensor is for instance 150 µm x 150 µm. Said depth of field and said field of view optically define a narrow sample volume. An adjustable lens may be used to adjust the position of the object plane, in which objects are sharply imaged on a sensor being arranged in functional relationship with an objective lens unit extending through or having a field of view directed through the optical aperture of the body for recording images in the direction of view. Said object plane might be chosen between the optical aperture and the reflector.

It is noted that within the framework of the present disclosure the use of the indefinite article "a" or "an" does in no way stipulate a singularity nor does it exclude the presence of a multitude of the named member or feature. It is thus to be read in the sense of "at least one" or "one or a multitude of".

The reflector may, in particular, be a mirror or a retroreflector. In particular, the use of a retroreflector together with light being emitted through the optical aperture has the advantage of being available as robust components and being tolerant with respect to the alignment. Mirrors can be produced by application of a suitable coating and/or polishing of suitable materials thereby avoiding the need to mount the reflector to e.g. a part of the body or a cantilevering strut or a similar means with which it is connected to the body. This can make the device more robust and avoid soiling.

In embodiments, the reflector is connected to the body by at least one strut. The strut can be a cantilevering strut. Preferably, a total circumferential extent of the at least one strut is no more than 180° . In embodiments, a total circumferential extent of the at least one stut may be no more than 90° , no more than 60° or no more than 30° . This allows a largely unhindered flow through or fluid exchange in the space between the reflector and the optical aperture. One single strut may be provided. The at least one strut may be releasably connected to the body. This would allow for instance a microscopy device which incorporates the herein claimed device for directing illumination to be used with or without the reflector. The reflector can be a reflective element attached directly to a cantilevering strut or a further structure, such as a bubble shield, can be attached to said strut and comprises or holds the reflector.

The distance between the reflector and the optical aperture may be adjustable, which would further increase versatility of the herein suggested subject matter.

In embodiments, a cross section of the body steadily increases along a distal to proximal direction along an optical axis of the optical aperture and the reflector. Said cross section increases from the optical aperture to the position of the maximal cross-section of the body. The cross sections are cross sections perpendicular to the optical axis.

In particular, the body can comprise at least two regions of positive curvature separated by a region with negative curvature. Preferably, a distal region of positive curvature is adjacent to or includes the optical aperture and the proximal region of positive curvature is adjacent to or includes the section of the body where it has its maximal cross section. Such shapes provide space between the distal front end and the vicinity of the reflector so that a fluid carrying the objects to be observed can easily enter the sample volume. At the same time, such shape provides sufficient internal volume to enclose at least some optical elements, such as lenses and parts of an illumination unit, allowing to use the device as part of a housing of a microscope. Preferably, the curvature is the gaussian curvature.

In embodiments, the device for directing illumination is suitable to be arranged in a streaming liquid to be measured and equipped with a bubble shield. This bubble shield comprises preferably a blunt body. The bubble shield has a cross section greater than the cross section of the optical aperture. The bubble shield is arranged distal of the distal front end of the body. Preferably, the reflector is part of the bubble shield. The bubble shield has preferably a positive curvature on its side facing the flow direction or the side facing away from the optical aperture.

In preferred embodiments, the streaming liquid to be measured has a flow direction and the bubble shield creates a wake when it is inserted in the streaming liquid. The device can be constructed such that the optical aperture is arranged in the wake during the intended operating conditions.

Many bioprocesses produce gases. In other cases, gases are introduced in the liquid to be observed by a mixing motion or on purpose. The gases can form bubbles which can stick to the walls of the vessel holding the liquid to be observed. Gas bubbles sticking to the optical aperture and /or the reflector can disturb the observations. Therefore, it is beneficial to avoid that they enter that volume between optical aperture and reflector. Gas bubbles typically move with the fluid stream and upwards, but due to their greater size, they typically do not follow small scale local flows. The bubble shield can create such a local flow field which entrains the objects to be observed, but not the bubbles and reduces thereby the risks of them disturbing the observations.

In a preferred embodiment, the cross section of the optical aperture is smaller than the cross section of the bubble shield. More preferably, the same is true for the effective cross-sections This ensures that the bubble shield is effective in protecting the whole optical aperture. The effective cross-section is preferably the cross section perpendicular to the flow direction during the intended operating conditions.

In a preferred embodiment, the cross section of the body is greater than the cross section of the optical aperture and essentially equal to the cross section of the bubble shield. More preferably, the same is true for the effective cross-sections as defined above. This embodiment ensures that the bubble shield is effective in protecting the whole optical aperture and avoids at the same time that the cross section of the device in total is greater than the one of the body. This facilitates, for example, the mounting of a microscope using the device. In addition, this embodiment can be combined with a body having a steadily increasing cross section along a distal to proximal direction along an optical axis of the optical aperture and the reflector, which, helps to provide space for the liquid to easily enter the sample volume and can create desirable, local currents.

In preferred embodiments, the bubble shield comprises a flow divider, which extends towards the optical aperture. Preferably, the reflector is mounted to said flow divider. In embodiments, the flow divider extends in the continuation of the flow direction in the wake of the distal part of the bubble shield during the intended operating conditions. In embodiments, the flow divider extends along the optical axis. When, during operation, the bubble shield creates a wake, the flow divider can divide the wake, creating a temporal steady flow and reducing the overall drag of the bubble shield.

In preferred embodiments, the flow divider has a conical shape with its radial extend decreasing along the distal-proximal direction. The flow divider reduces the volume in the wake which has a very low local flow velocity and ensures thereby the desired flow of particles to be observed through the field of view of a microscope using the device. The conical shape supports the desired local vortices which bring the fluid to be measures to the sampling region. In addition, the flow divider offers a convenient place to mount the reflector: it is preferably on the tip of the conical shape, i.e. at its most proximal end.

In embodiments, the bubble shield, preferably comprising the blunt body and/or the flow divider, is rotationally symmetric with the optical axis being the axis of symmetry. This symmetry might be broken by a strut with which the bubble shield may be connected to the body of the device.

The herein suggested device for directing illumination may be used together with an objective lens unit, such as an objective lens unit for a microscope. The device and the objective lens unit can form an assembly. The objective lens unit comprises an enclosure having a distal front end. A front end optical aperture of the objective lens unit is provided in the distal front end of the objective lens unit. The objective lens unit further comprises an objective lens system arranged inside the enclosure and proximal from the front end optical aperture of the objective lens unit. The objective lens system is configured to collect light received through the front end optical aperture of the objective lens unit. The device for directing illumination is attached to the objective lens unit. The device for directing illumination is arranged such that the reflector is arranged distal from the distal front end of the objective lens unit and a non-zero distance from the front end optical aperture of the objective lens unit. It is arranged to reflect at least part of the light received from the front end of the body back into the front end optical aperture of the objective lens unit. Preferably, the light received by the reflector is emitted through the front end optical aperture of the objective lens unit as well as through the front end of the body. The skilled person will appreciate that, implicitly, the reflector is in the field of view of the objective lens unit. The objective lens unit allows to observe a sample volume located between the front end optical aperture of the objective lens unit and the reflector, and in particular embodiments through the optical aperture of the body of the device for directing illumination.

The objective lens unit may in particular be suitable to be used when at least partially submerged in a liquid, for instance a sample liquid with suspended biological cells for cytometry. In particular, the device for directing illumination and/or the objective lens unit or the combination of both can be liquid-proof. This makes the assembly suitable to be used when it is at least partially submerged in a liquid. For example, a ring-shaped body could be attached to liquid-proof objective lens unit, or a liquid-proof cap could be the body and, at the same time, enclose at least parts of an objective lens unit which are not liquid-proof, making the combination liquid-proof. In further embodiments, a liquid proof body could be mounted to the container of the liquid to be observed, such as a bioreactor, and thereby creating a liquid-proof barrier between the inside of said bioreactor and the objective lens system.

In another aspect, disclosed is an objective lens unit for a microscope. The objective lens unit may in particular be suitable to be used when at least partially submerged in a liquid. Preferably, the liquid is a sample liquid comprising the objects to be observed. The objective lens unit comprises an enclosure. At least a part of the enclosure is formed by the body of a device for directing illumination of the type outlined above. The front end of the body of the device for directing illumination is configured and arranged to provide a distal front end of the enclosure. The optical aperture of the body of the device for directing illumination provides a front end optical aperture of the objective lens unit. The reflector of the device for directing illumination is connected to the enclosure. The reflector is arranged distal from the front end of the enclosure and a non-zero distance from the front end optical aperture of the objective lens unit. It is configured to receive light emitted through the distal front end of the enclosure and to reflect at least part of the light received from the distal front end back into the front end optical aperture. The objective lens unit further comprises an objective lens system arranged inside the enclosure and proximal from the front end optical aperture of the objective lens unit and configured to collect light received through the front end optical aperture.

In more specific embodiments of the above-described objective lens unit, the enclosure comprises a cap and a sleeve, wherein the objective lens system is provided inside the sleeve. The cap is provided by the body of the device for directing illumination. The cap comprises a lateral sheath, a front wall and a rear port. The front end optical aperture of the objective lens unit is provided in the front wall of the cap. The sleeve is preferably at least partially received inside the cap, in particular through the rear port of the cap. In embodiments, the cap is at least partially received inside the sleeve, in particular the objective lens system is received through the rear port of the cap while the lateral sheath contacts the inside of a housing defining the outside of the sleeve. In embodiments, the sleeve may be threadedly received inside the cap or the cap may be threadedly received inside the sleeve. A sealing, for instance provided by an O-ring, may be provided between the cap and the sleeve. In other embodiments, the sleeve may simply be plugged into the cap, wherein the sleeve and the cap may be equipped with centring means such that the optical axis of the front end optical aperture of the objective lens unit and of the objective lens system coincide when the sleeve is received inside the cap. The cap may for instance be fixed to a bioreactor and extend through a port of the bioreactor, with a distal front section of the lateral sheath and the front wall positioned inside the bioreactor and the rear port of the cap being accessible outside the bioreactor. The sleeve may successively be plugged into different caps so as to be used in different bioreactors or at different locations of one bioreactor. The rear port of the cap and the sleeve can be equipped with structures allowing to secure the sleeve to the cap. For example, the cap can be equipped with threads, radial pegs or slots and the sleeve can comprise a collar and a union nut with corresponding threads, slots or radial pegs allowing the formation of a threaded connection or a bayonet mount.

Generally, the objective lens unit or the body may comprise an immersion lens arranged and configured to collect light entering the objective lens unit through the front end optical aperture of the objective lens unit respectively the body through the optical aperture. In embodiments, the immersion lens may be arranged inside the respective optical aperture and close said optical aperture. In other embodiments, however, the immersion lens may be arranged proximal from the optical aperture, that is, between the optical aperture and the objective lens system in the assembled state. The immersion lens might be a solid immersion lens, so-called SIL, but may in other instances comprise a liquid immersion lens. The front end optical aperture may be closed by a window as the most distal optical component. The liquid immersion lens, in this case, is provided with a gap between the window and the liquid immersion lens. The gap is filled with a liquid. The material of the window, the liquid, and the immersion lens are chosen such that the refractive indices match well-defined relations, wherein the shape and focal length of the immersion lens may further be considered. Persons skilled in the art of microscopy and/or optical engineering are perfectly familiar with the appropriate choice of materials and design. Solid immersion lenses generally have the shape of truncated spheres, wherein they most commonly comprise one half of a sphere, so called hemispherical SILs, or more than half of a sphere, so-called Weierstrass SILs, also referred to as superhemispherical SILs or superSILs.

Objective lens units incorporating immersion lenses have a relatively narrow depth of field, typically measuring only some µm along an axial direction or direction of view. The position of the object plane, in which objects are sharply imaged on a sensor being arranged in functional relationship with the objective lens unit for recording images, in the direction of view, strongly varies with tolerances of the objective lens unit, i.e., in particular the relative positions of the immersion lens and the objective lens system. Tolerances of the position of the object plane may be found unsuitable if manufacturing and assembly tolerances of the objective lens unit are not restricted to a micrometer range, which turns manufacturing expensive and unsuitable for series manufacturing. In addition, objective lens units can be exposed to thermal or mechanical stress during use. For example, an objective lens unit used in a bioprocess might need to be subjected to an autoclave treatment or "sterilization in place" (SIP) or "clean in place" (CIP) procedures. The sterilization for example, is usually carried out by means of superheated steam, for example with steam at 120° C and 2 bar over a period of about 60 to 70 minutes whereby the exact parameters may vary depending on the application and the system. The stresses caused by such treatments but also during the intended use as well as aging, can cause small deformations and shifts of the components relative to each other. It might thus be found advantageous if the objective lens system comprises at least one adjustable lens. The adjustable lens might be the most distal optical element of the objective lens system, i.e., may be closest to the immersion lens. For a number of reasons, including avoiding vibrations and autoclavability, the adjustable lens might be motorless adjustable. In embodiments, the adjustable lens might be axially shiftable relative to the immersion lens by a piezo actuator. In other embodiments, the adjustable lens might be a tunable lens, i.e., a lens which can controllably change its focal length, for instance in that the curvature of the optically active surfaces is changed. Such tunable lenses are available off-shelf. The position of the object plane may thus be adjusted. The position of said object plane might be chosen to be between the optical aperture of the body or the objective lens unit and the reflector.

The front end optical aperture of the objective lens unit or the optical aperture of the body of a device for directing illumination may in embodiments be closed by one of a window and an immersion lens, and may moreover be closed in a liquid-proof manner. Thus, the objective lens unit, device or assembly comprising the objective lens unit, in these embodiments is suitable for use when at least partially submerged in a liquid. The liquid is preferably a sample liquid in which the objects to be observed are suspended. At least a distal or front section of the objective lens system, including the front end optical aperture or at least a distal front end of the body including the optical aperture, is thus suitable for being submerged in a liquid. Further, at least the objective lens system or the device for directing illumination, and in embodiments the entire objective lens unit or assembly comprising the objective lens unit, might be autoclavable or suitable to be subjected to "Sterilization in Place" (SIP) or "clean in place" (CIP) procedures. In order for the objective lens unit or an assembly comprising the objective lens unit to be suitable to be used when at least partially submerged in a liquid, at least a distal section of the objective lens unit or the body might be liquid-proof.

In more specific embodiments, the objective lens unit or the body comprises a proximal rear connector interface, wherein at least a section of the objective lens unit or the body excluding the rear connector interface is liquid-proof. In specific embodiments, the body is a cap and the rear connector interface is the rear port of the cap and the lateral sheath and the front wall form a liquid proof container with the only opening being the rear port. In further specific embodiments, the objective lens unit comprises an interface to which an illumination source and/or an optical sensor can be functionally coupled, and it is this interface which is the rear connector interface. Preferably, the optical sensor is a 2D-optical sensor such as a CCD or CMOS sensor and it is most preferable provided as part of a camera.

In specific embodiments, an illumination source and/or an optical sensor are integrated in the objective lens unit and the objective lens unit comprises an interface which allows to receive and transmit data and/or power. Preferably, such an objective lens unit is liquid-proof. For example, data can be transmitted wirelessly, and power can be transmitted via induction. In other examples a cable connection, coupling electrical and/or optical transmission lines to the objective lens unit. In other embodiments, the interface which allows to receive and transmit data and/or power is a rear connector interface.

The most distal optical element of the objective lens unit or the body, e.g., in particular a window or the immersion lens closing the front end optical aperture of the objective lens unit respectively the front end of the body, may be flush with a distal outer surface of the objective lens unit, or of the enclosure, or of the distal front end of the body respectively. This results in a smooth distal tip of the objective lens unit or the body, which increases robustness of the objective lens unit and significantly reduces the risk of building up dirt depositions or biofouling and reduces vortices around the distal tip of the objective lens unit when placed in a liquid flow. The distal front end or tip of the objective lens unit or the distal front end of the body may in this respect be flat or convexly shaped.

A circumferential outer wall of the objective lens unit, the assembly comprising the objective lens unit or the body may be circular-cylindrical with an outer diameter of 25 mm or less and in particular 12 mm or less. Preferably, the device for directing illumination is shaped such that, in at least one orientation, it could be enclosed by a circular cylinder with an outer diameter of 25 mm or less and in particular 12 mm or less. This allows the objective lens unit to be inserted into a bioreactor or vessel through standard ports, such as Ingold standard access ports and Eldon-James ports. In embodiments, it is a distal part of the objective lens unit, the assembly and/or the body which, in at least one orientation, fits in the volume of a circular cylinder with an outer diameter of 25 mm or less and in particular 12 mm or less while a proximal part extends at least partially in radial direction over said cylinder. This proximal part could comprise a collar or mounting means which could facilitation mounting of further components such as cables or data transmitters and it could prevent that the objective lens unit, the assembly and/or the body is inserted by more than the desired amount in the bioreactor or vessel.

The device for directing illumination, the assembly or the objective lens unit described above may further comprise a sample illumination unit. The sample illumination unit comprises an illumination source. The sample illumination unit comprises further a means for coupling light from the illumination source into the objective lens unit or into the body.

In particular embodiments, the means for coupling light from the illumination source into the objective lens unit is arranged distal from the objective lens system and proximal from the front end optical aperture of the objective lens unit and is further configured to project said light into a proximal-distal direction of the objective lens unit through the front end optical aperture and onto the reflector.

In embodiments comprising an immersion lens, the means for coupling light from the illumination source into the objective lens unit or into the body may be arranged distal from the objective lens system and proximal from the immersion lens.

The illumination source may be functionally coupled to the means for coupling light from the illumination source into the objective lens unit or into the body and may be placed laterally from the optical path of the objective lens unit. The means for coupling light from the illumination source into the objective lens unit may be a beam splitter arranged and configured to reflect part of the light form a laterally arranged illumination source. For example, an illumination source which is arranged off-axis relative to the optical axis of the objective lens unit and outside the detection optical path of the objective lens system, into a distal direction of the objective lens unit and towards and through the front end optical aperture of the objective lens unit and/or through the optical aperture of the body.

In other embodiments, the illumination source is arranged in additional apertures provided in the objective lens unit or the body or the light from the illumination source is guided to such addition apertures, for example with light guides, fibre optics or suitable optical elements such as reflecting surfaces. In other embodiments, however, for instance fiber optics or other light guides alone or in combination with further optical elements may be applied to couple light from the illumination source into the objective lens unit or into the body.

The illumination source may be or comprise an LED. The illumination source may in embodiments be a monochromatic light source or a light source having a narrow half power bandwidth of, for instance, 50 nm or less, 20 nm or less, or 10 nm or less. Monochromatic or narrow bandwidth imaging results in avoiding chromatic aberration when imaging objects, and thus to further increase image quality. This might be achieved in emitting monochromatic or low bandwidth light from the illumination source, using filters, or a combination thereof. In other instances, a bandpath filter may be used at a suitable location of an instrument.

However, in certain applications it might be useful to capture pictures at at least two sufficiently different wavelength, thus using chromatic aberration to simultaneously retrieve images from at least two different object planes. Thus for instance a broadband or multi-band light source or a multitude of light sources emitting light with different spectra may be used. For a non-limiting instance, a broadband LED or a multitude of LEDs emitting light at different peak wavelengths may be used.

In aspects, the reflector is part of a reflector assembly. The reflector assembly comprises a reflector attached to at least one strut. The at least one strut is configured to be mounted to the enclosure of an objective lens unit, for example to a cap suitable to be attached to a sleeve of an objective lens unit. The reflector assembly is sized and shaped such that the reflector is positioned a non-zero distance distal from a front end of the enclosure of the objective lens unit and is positioned and configured to receive light emitted through the front end of the objective lens unit and to reflect at least part of the light received back into the optical aperture when the reflector assembly is attached to the enclosure of the objective lens unit. Preferably, the light received by the reflector is emitted through the front end optical aperture provided in the front end of the objective lens unit. The strut can be a cantilevering strut to which the reflector, being a reflective element, is directly mounted. A further structure, such as a bubble shield, can be attached to the strut and comprise or hold the reflector. In this case, the reflector assembly comprises the reflector, the strut and the further structure.

In still another aspect, disclosed is a microscope for in-situ application, preferably for measurements inside a bioreactor. The microscope comprises an objective lens unit or an assembly comprising an objective lens unit of any kind described above and an optical sensor. The optical sensor is functionally coupled to the objective lens unit to receive light transmitted from a front side of the objective lens unit and through the objective lens unit. In particular, the optical sensor is functionally coupled to the objective lens unit to receive light transmitted through the front end optical aperture and through the objective lens system. In particular aspects, the microscope may be configured for attaching it to a vessel, such as a bioreactor, comprising a standard port, wherein the microscope is further configured such that at least a distal section of the objective lens unit or the body, i.e., in particular the section comprising the optical aperture, may be positioned inside the vessel, such as a bioreactor, whereas a microscope head and/or the rear connector interface may be located outside the bioreactor. Preferably, the microscope head or the rear connector interface comprises the interface to receive and transmit data and/or power. The optical sensor may in particular comprise an image sensor and more in particular a CCD or CMOS image sensor. The optical sensor may be arranged in a microscope head or may be integrated in the objective lens unit.

The microscope may further include a sample illumination unit comprising an illumination source and a means for coupling light from the illumination source into the objective lens unit. The sample illumination unit may in instances be arranged proximal from the objective lens unit, for instance in a microscope head, and/or it may be integrated in the objective lens unit, as described above or be coupled to the rear connector interface. It is suggested that a sample illumination unit being arranged in the objective lens unit and coupling the light from the illumination source into the objective lens unit distal from the objective lens system provides most efficient use of light from the illumination source. The optical path of the light emitted from the sample illumination unit and the optical path of the light collected from the objects in the sample volume may coincide at least along sections of the respective optical paths. The possible spectral characteristics of the illumination source of the illumination sources, respectively, and the effects they yield, are discussed above. The microscope may provide means to split the light along an optical path from the front end optical aperture to the sensor, such as beam splitters. Elements which allow to select different narrow light wavelength ranges, such as for instance, while not limited to, bandpass filters, may be arranged in the thus generated different optical paths. Images at different light wavelengths may thus be processed. When applying a broadband or multi-colour light source to illuminate the sample volume, and the different wavelengths are sufficiently far apart, the images at different wavelengths originate from different object planes due to chromatic aberration. In still further embodiments, the optical path may be divided, for instance using beam splitters, and different lenses might be arranged in the different optical paths so as to image different object planes on a sensor through the different optical paths.

In still further aspects, methods for performing cytometry are suggested. A method comprises using a microscope as described above. The method further comprises submerging at least a distalmost front section of the objective lens unit, including the distal front end and the front end optical aperture, or the distal front end of the body including the optical aperture, in a sample liquid. Preferably, the sample liquid is contained inside a bioreactor. Light is emitted through the front end of the objective lens unit or the body and is directed in a direction towards the reflector. At least a part of the light that hits the reflector is reflected back to the front end optical aperture of the objective lens unit and/or the optical aperture of the body. This light passes through a sample volume of the sample liquid. At least one image generated by the objective lens unit is recorded using the optical sensor. Preferably, in the case of a specific illumination by light travelling from the front end optical aperture of the objective lens unit to the reflector and light reflected from the reflector back to the front end optical aperture of the objective lens unit, the at least one image includes forward scattered light and backward scattered light from objects, such as biological cells, contained in the sample volume. A similar situation occurs if the illuminating light reaches the reflector in a small angle with respect to the optical axis. Such a small angle preferably occurs if the light from the illumination source passes through additional apertures close to but not necessarily identical to the optical aperture or where the light from the illumination source does not follow the path of the reflected light.

Preferably, the method comprises further the step of inserting at least a distal section of the objective lens unit or of the assembly comprising the objective lens unit through a port of a vessel, for example a bioreactor.

The method may further comprise emitting the light in pulses, wherein one pulse is emitted per frame recorded by the optical sensor, and wherein a pulse duration is in particular 20 µs or less. The duration of the light pulses may be 10 µs or less. The resulting short illumination times enable to capture clear images even when the cells in the sample volume move. On the other hand, blurredness of the images with longer illumination time may be used to determine flow velocity through the sample volume.

The method may further comprise emitting light in pulses wherein a pulse duration is 20 µs or less, preferably 10 µs or less. Pulsed operation of the illumination source with short, microsecond, pulse durations enable to obtain the desired stroboscopic effect independent from the frame rate recorded by the sensor.

In further embodiments, multiple pulses are emitted per frame recorded by the optical sensor, resulting in images showing a plurality of pictures of the same object on the same frame if the pulse rate is suitable chosen, depending on speed of the particles and the recorded field of view. This allows to measure said speed and, if the object rotate, to image it from different sides.

The method may further include the step of setting a focal length of the objective lens unit such that objects in a sample volume located at a desired position between the front end optical aperture of the objective lens unit and the reflector are sharply imaged on the optical sensor. This may involve actuation of an adjustable lens, which is in particular a tunable lens, as described above. The desired position can be defined with respect to targets provided on an optical element arranged on the optical axis: For example, the adjustable lens is adjusted such that said targets are sharply imaged followed by adjusting the adjustable lens by a predetermined adjustment magnitude, causing the motion of the position of the volume in which objects are sharply imaged to the desired position.

If the objective lens unit, or the assembly comprising the objective lens unit, is an objective lens with an enclosure comprising a cap and a sleeve, as outlined above, the method may further comprise inserting at least a front section of the cap through the port of a bioreactor and sealing the port of the bioreactor with the cap. The rear port of the cap is maintained outside the bioreactor. At least a distalmost front section of the cap including the front wall with the optical aperture is submerged in a sample liquid inside the bioreactor. The sleeve comprising the objective lens system is inserted into the cap.

The method may further comprise the step of setting a focal length of the objective lens unit such that the reflector is imaged on the optical sensor. In this embodiment, a warning is issued, if the observed reflectivity is lower than an expected value and/or if the image shows unexpected structures. A reduction in the reflectivity or structures which appear over time can be an indication of biofouling, soiling or degrading optical surfaces. To minimize the risks of false warnings, the method can further comprise the step of evaluating observations of the sample volume before and/or after imaging the reflector and estimating the object or cell density on these images and considering the appearance and/or density of the observed objects or cells in setting the expected value. Further, it is possible to issue a warning only after multiple images of the reflector show a consistent trend, indicating a loss of imaging quality, such as biofouling, soiling or aging effects.

It is understood that the features and embodiments disclosed above may be combined with each other. It will further be appreciated that further embodiments are conceivable within the scope of the present disclosure and the claimed subject matter which are obvious and apparent to the skilled person by virtue of the present disclosure.

### Brief description of drawings

- Fig. 1: A microscope arranged for in-situ cytometry inside a bioreactor;
- Fig. 2: a first exemplary embodiment of an objective lens unit suitable for in-situ cytometry;
- Fig. 3: a second exemplary embodiment of an objective lens unit suitable for in-situ cytometry;
- Fig. 4: a detail of an exemplary embodiment of the arrangement of an immersion lens and front end optical aperture of an objective lens unit suitable for in-situ cytometry;
- Fig. 5: an objective lens unit of the type herein suggested, which includes a sample illumination unit and a reflector arranged distal from the front end optical aperture;
- Fig. 6: a further possible arrangement of a reflector distal from the front end optical aperture;
- Fig. 7: an exemplary embodiment of an objective lens unit having a different tip geometry; and
- Fig. 8: a view along an optical axis of an objective lens unit having targets.
- Fig.9a: A first example of an objective lens unit comprising a reflector and a bubble shield
- Fig.9b: A second example of an objective lens unit comprising a reflector and a bubble shield
- Fig. 10a: An embodiment of a reflector assembly in a cross-sectional view
- Fig. 10b: An embodiment of a reflector assembly in a perspective view

It is understood that the drawings are partly schematic, and details not required for instruction purposes may have been omitted for the ease of understanding and depiction. It is further understood that the drawings show only selected, illustrative embodiments, and embodiments not shown may still be well within the scope of the herein disclosed and/or claimed subject matter.

### Description of embodiments

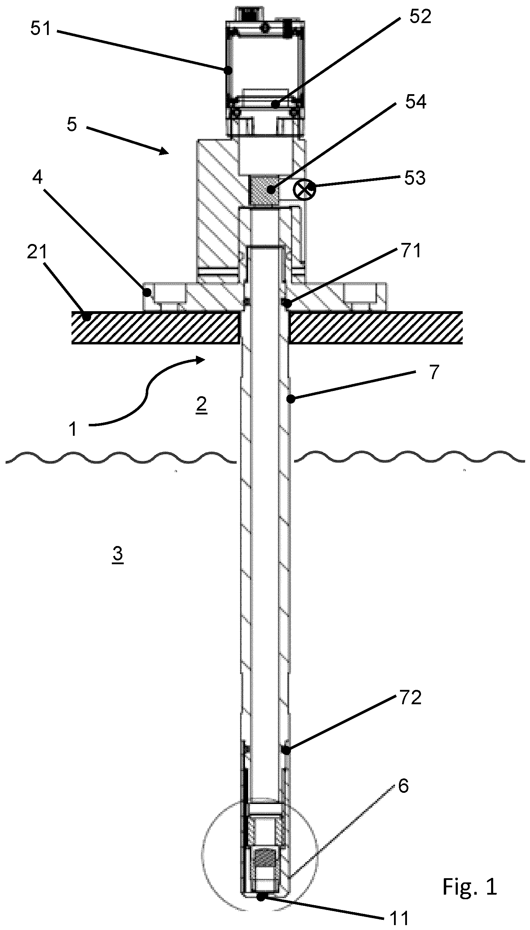

Figure 1 shows a microscope 1 mounted to wall 21 of tank 2 and extending through wall 21 into tank 2 and into sample liquid 3 contained in tank 2. Microscope 1 is attached to wall 21 of tank 2 by flange 4. Head 5 of microscope 1 is arranged outside tank 2. Camera 51 is mounted at a proximal end of microscope 1 and provides optical sensor 52. A distal end 11 of microscope 1 is provided by objective lens unit 6. Objective lens unit 6 and head 5 are connected by hollow shaft 7. In embodiments, the hollow shaft 7 can be part of the enclosure of the objective lens unit 6. O-ring sealings 71 and 72 are provided at the respective connections between shaft 7 and head 5 and shaft 7 and objective lens unit 6, respectively. Light collected by objective lens unit 6 at distal end 11 of microscope 1 passes along an optical path through hollow shaft 7 and is projected onto optical sensor 52 of camera 51. Optical sensor 52 and camera 51 are configured to capture or record light, or an image of an object, projected on optical sensor 52. Illumination source 53 is in this embodiment mounted to head 5 lateral from the optical path. Beam splitter 54 is arranged inside head 5 in in the optical path of light from objective lens unit 6 to optical sensor 52. Beam splitter 54 serves as a means for coupling light from illumination source 53 into objective lens unit 6 and deflects part of the light from illumination source 53 towards objective lens unit 6, where the light exits microscope 1 at distal end 11 and illuminates a sample volume of liquid 3. In further embodiments, light guides such as optical fibers are coupled to the illumination source 53 and guides the light such that it exits microscope 1 at the distal end 11 and illuminates the sample volume of liquid 3. The beam splitter 54 can be omitted in these embodiments. Light from the thus illuminated sample volume of liquid 3 enters microscope 1 at distal end 11 thereof and travels through objective lens unit 6 and hollow shaft 7 towards optical sensor 52. The light travelling back is partially deviated by beam splitter 54, if it is present, while another share of the light from the sample volume travels to optical sensor 52 and is captured by optical sensor 52. Thus, magnified views of objects in liquid 3 can be imaged and images can be recorded by camera 51. The illumination and image optical paths coincide in the illustrated embodiment in large sections of the setup, that is, in the present depiction, below beam splitter 54. In other embodiments, the illumination and image optical paths are separate from each other of coincide only in the vicinity of distal end 11. Tank 2 might be a bioreactor. Liquid 3 might hence contain biological cells as objects to be observed. Cells might be observed in situ by use of the shown microscope 1. Microscope 1 thus enables in-situ cytometry. Illumination source 53 might be pulsed. One single pulse of typically some 10 µs duration might be emitted by illumination source 53 per frame captured by camera 51. Hence, clear and sharp images of even strongly magnified objects may be obtained, even if the objects move. On the other hand, longer illumination times or multiple illumination pulses per frame recorded by camera 51 may assist in determining flow velocity and direction through the sample volume.

Light source 53 might emit monochromatic light or light at a narrow bandwidth to avoid chromatic aberration effects. Alternatively, a bandpass optical filter might be arranged in the illumination and/or imaging light path. In other embodiments, the sample volume might be illuminated by a broadband light source or a multi-colour light source, and optical sensor 52 might receive light at different wavelengths. Due to chromatic aberration, the distance of the object plane from which a sharp image is received on optical sensor 52 from the distal end 11 of microscope 1 is different for the different wavelengths, or colours. Thus, if light with sufficiently different wavelengths is used, some kind of 3D information of objects might be obtained.

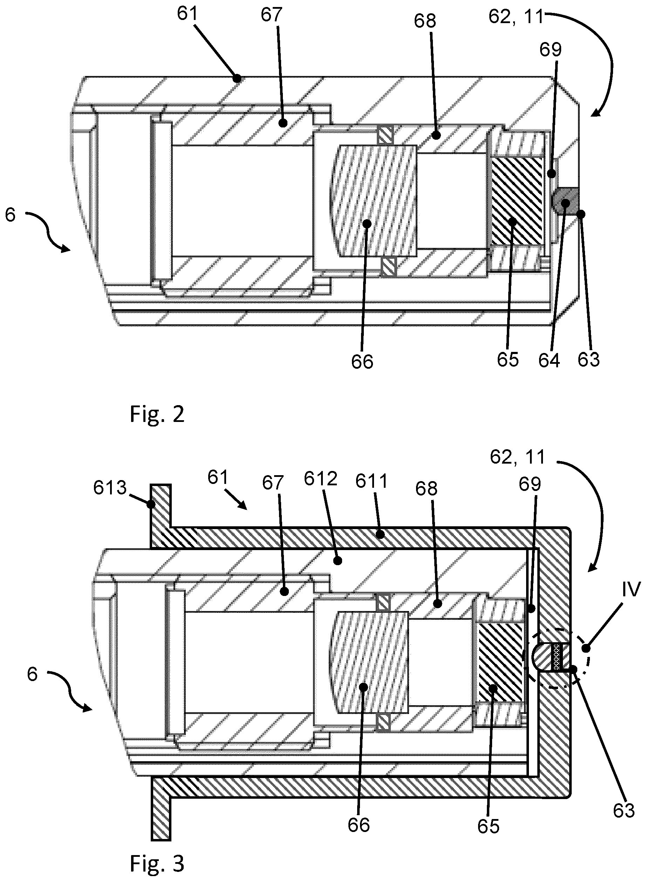

Figure 2 shows a more detailed view of objective lens unit 6. Exemplarily shown embodiment of objective lens unit 6 comprises enclosure 61 including optical components 64, 65 and 66. Optical aperture 63 is provided through enclosure 61 at a distal front end 62 of objective lens unit 6, or distal end 11 of microscope 1, respectively. Light from illumination source 53 may exit objective lens unit 6 through optical aperture 63 and light from a sample volume may enter objective lens unit 6 through optical aperture 63. A solid immersion lens 64 is arranged inside optical aperture 63 to receive light through front end optical aperture 63. Solid immersion lens 64 yields multiple purposes. On the one hand, a solid immersion lens provides a high numerical aperture with a comparatively small entrance area, which in turn reduces noise. On the other hand, objectives incorporating immersion lenses as a front lens have a narrow depth of field. Typically, the depth of field which can be sharply imaged on an optical sensor measures some µm. The field of view captured by a suitable sensor might for instance be 150 µm x 150 µm. Thus, a very narrow sample volume is defined optically and needs not to be mechanically delimited. The narrow depth of field largely serves to avoid that cells overlapping each other in the direction of view are captured in an image and thus serves to avoid interferences of overlapping cells. Optical components 65 and 66 jointly form an objective lens system arranged to collect light received by immersion lens 64 through front end optical aperture 63. A disadvantage of using immersion lenses at the front end 62 of objective lens unit 6 is that position tolerances of the arrangement of optical components 65 and 66 relative to immersion lens 64 translate into a large variance of the position of the object plane which is sharply imaged on the optical sensor, and hence of the position of the sample volume. DE 10 2015 014110 for instance states that 30 µm precision is required to define the object plane with about 3 µm accuracy. This, however, turns manufacturing such objective lens units with immersion lenses units expensive and makes such prior art solutions sensitive to changing conditions such as aging and temperature variations.

The herein suggested subject matter mitigates these issues. Lens 65 can be a motorless automated adjustable lens. In embodiments, as in the shown example, a tunable lens 65 is used. It was shown that with off-the-shelf tunable lenses manufacturing and assembly tolerances of 1 mm and more may be compensated by the change of focal length of the tunable lens 65. In other embodiments, the adjustable lens might be displaceable along the axis of the objective lens unit by a piezo actuator. Both exemplary solutions can be implemented inside the compact enclosure of the objective lens unit 6, are autoclavable and/or can withstand SIP or CIP procedures and may operate without vibrations. Tunable lens 65 is provided next to immersion lens 64, with a small air gap 69 between tunable lens 65 and immersion lens 64 such that deformation of the tunable lens surface is not impeded by immersion lens 64. Lens 66 is arranged proximal of tunable lens 65 and is for instance an aspheric lens. Lenses 65 and 66 are held in place by threaded sleeve 67, which is screwed into enclosure 61. A distance sleeve 68 is shown as a means to maintain the distance between aspheric lens 66 and tunable lens 65. The distal or front surface of immersion lens 64 is flush with an outer surface of a distal front wall of enclosure 61. Thus, vortices in the liquid flow are avoided, less dirt and biofouling can accumulate and objective lens unit 6 is easier to clean.

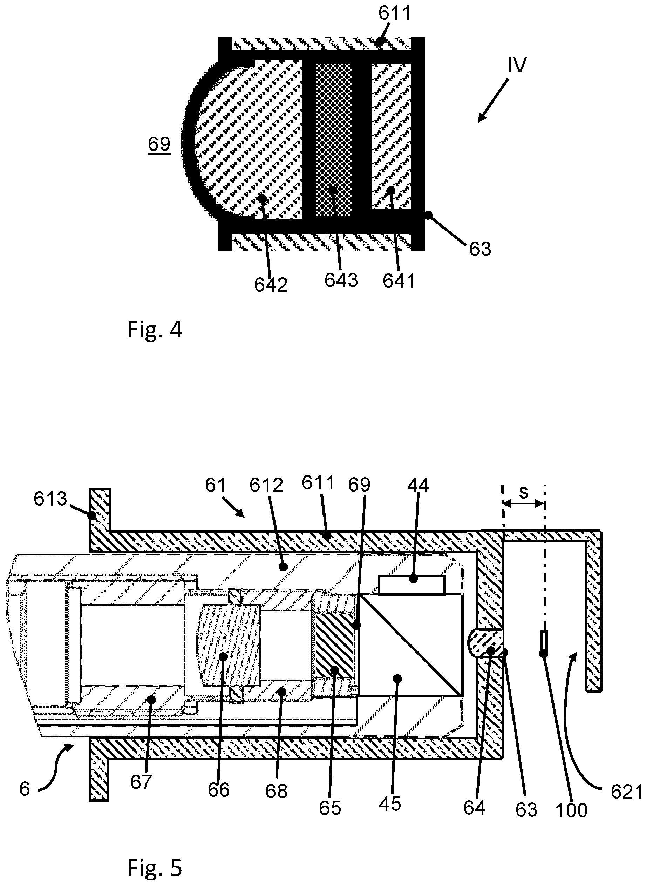

In another embodiment shown in figure 3, enclosure 61 comprises cap 611 and sleeve 612. Cap 611 comprises a distal front wall and a lateral sheath. A flange 613 extends radially outward at a proximal, rear end of the lateral sheath. The objective lens system 6, comprising tunable lens 65 and aspherical lens 66, is provided inside sleeve 612. Generally, the assembly of the objective lens system is similar to that outlined in connection with figure 2. Sleeve 612 with the optical lens system provided therein may be inserted into cap 611 through a rear port of cap 611. Optical aperture 63 is provided in the distal front wall of cap 611. Turning to figure 4, detail IV is discussed. Plan window 641 closes optical aperture 63 distally and is provided flush with a distal outer surface of the front wall of cap 611. Liquid immersion lens 642 is provided proximal to window 641 with a gap 643 provided between liquid immersion lens 642 and window 641. Gap 643 is filled with an immersion liquid, such as oil, water, silicon or the like. The refractive indices of window 641, liquid immersion lens 642 and the immersion liquid inside gap 643 and the geometry of liquid immersion lens 642 are chosen to match well-defined relations, well-known to a person skilled in the art of microscopy and/or optical engineering. In still other embodiments, a solid immersion lens may be cemented to a distally arranged window distally closing the optical aperture. It is noted that these exemplary immersion lens assemblies may, of course, also be used in connection with an enclosure as shown in figure 2.

As is also seen in figure 4, air gap 69 is provided proximally adjacent immersion lens 642. Turning again to figure 3, it is seen that tunable lens 65 is provided next to immersion lens 642. Sleeve 612 with the objective lens system may be functionally connected to an optical sensor at its proximal end such that an image transmitted through objective lens unit 6 may be captured by the optical sensor. For a non-limiting instance, a proximal end of sleeve 612 may be connected to a shaft 7, which in turn is connected to a microscope head 5, as shown in figure 1. In other instances, the microscope head 5 may be directly connected to the proximal end of sleeve 612. A distal section of cap 611 may be inserted into a vessel such as a tank 2, for example a bioreactor, through a port thereof and may be submerged in a sample liquid. Flange 613 remains outside the bioreactor and serves to fix cap 611 to the vessel, for example the bioreactor. A rear port of cap 611 thus is provided outside the bioreactor. Cap 611 is, apart from the rear port, liquid-proof. Sleeve 612 with an optical sensor functionally connected thereto may be inserted into cap 611 from outside the vessel, such as the bioreactor, to jointly form a microscope suitable for in situ cytometry. The objective lens system and all components attached thereto are protected from contact with the liquid inside the vessel, such as the bioreactor. Therefore, they can be exchanged and/or removed without influencing the contents inside the vessel.

An objective lens unit 6 comprising the features of the herein claimed invention is illustrated in figure 5. An illumination unit comprising illumination source 44 and beam splitter 45 as a means for coupling light from illumination source 44 into objective lens unit 6 is provided in objective lens unit 6. Beam splitter 45 is arranged in the optical path of objective lens unit 6, while illumination source 44 is arranged lateral of the optical path. As will be appreciated, beam splitter 45 deflects part of the light from illumination source 44 in a forward or distal direction of objective lens unit 6 and towards immersion lens 64. Light is emitted through front end optical aperture 63 in a forward or distal direction of objective lens unit 6. Objective lens unit, through tunable lens 65, is adjusted such that an object plane is located a distance s distal to front end optical aperture 63. A sample volume 100 is thus sharply imaged on an optical sensor functionally coupled to objective lens unit 6 and proximal from objective lens unit 6. The typical size of sample volume 100 is discussed above. However, back scatter from the biological cells in the sample volume may generally be low due to the small size and low difference in refractive index between the cells and the liquid in which the cells are suspended. Thus, a reflector 621 is provided cantilevering from a cap 611, which encloses the sleeve 612 of optical lens unit 6. The reflector 621 is arranged on the optical axis of objective lens unit 6. Reflector 621 may for instance be a mirror or a retroreflector. In the illustrated embodiment, reflector 621, the cap 611 and the cantilevering strut form a reflector assembly which is attachable to the objective lens unit 6. It is understood that also reflector 621 together with a cantilevering strut might be a reflector assembly which might be screwed or otherwise mounted to enclosure 61 of optical lens unit 6. Reflector 621 is arranged such that at least a part of the light emitted through front end optical aperture 63 is reflected back into front end optical aperture 63. Said reflected light passes through sample volume 100. Biological cells contained in sample volume 100 are thus illuminated from distal. Hence, forward scattered light from objects, such as biological cells or other particles, in sample volume 100 is received by objective lens unit 6 and imaged on an optical sensor functionally connected to the rear or proximal end of objective lens unit 6. The intensity of forward scattered light may be orders of magnitude higher than that of the backward scattered light. Consequently, the forward scattered light dominates the backward scattered light in these images on the optical sensor. This allows to improve the quality of the cytometry using the strong signal obtained by transmission microscopy. At the same time, there is no need of a light source inside the liquid to be observed or for means to remove the reflectance image, such as polarisation filers, providing thereby a particular robust and compact illumination system and microscope.

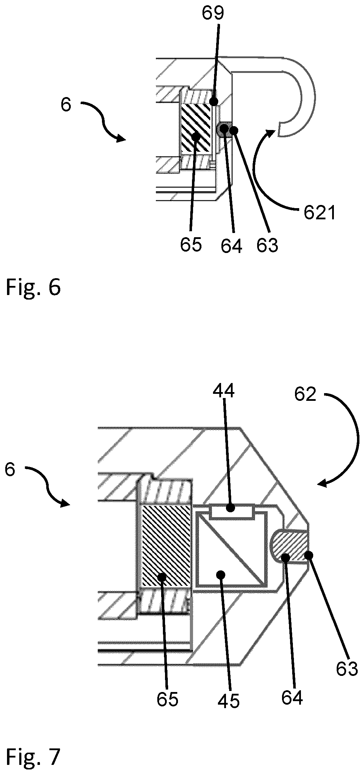

As illustrated in figure 6, reflector 621 may for another non-limiting instance also be provided on a U-shaped carrier. This can minimize the influence on flow of the liquid to be observed. In any embodiments, it may moreover be provided that the distance between forward optical aperture 63 and reflector 621 may be varied.

Figure 7 illustrates an embodiment in which distal front end 62 of objective lens unit 6 is not flat, as shown in the examples of figures 2 through 6, but is generally convex, for instance conical. For other non-limiting instances, the distal front end 62 of objective lens unit 6 may be rounded, for instance dome-shaped. A dome-shaped or a convex shape can help to minimize the impact on the flow of the liquid to be observed and can help to reduce the likelihood of gas bubbles sticking to the distal front end 62.

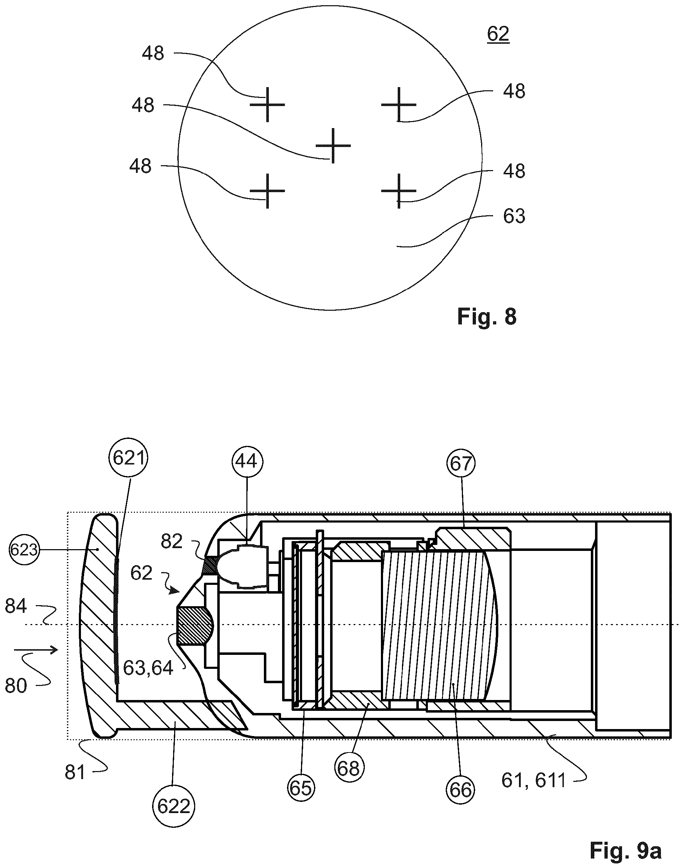

Figure 8 shows a plan view onto distal or front end 62 of objective lens unit 6. In embodiments of the herein disclosed subject matter, one or more optically detectable targets 48 may be provided on a distal surface of the optical component distally closing front end optical aperture 63, e.g. solid immersion lens 64 of figures 2, 5, 6 and 7 or window 641 of the embodiment illustrated in figures 3 and 4. In a mode of operating a microscope including said objective lens unit, tunable lens 64 or other motorless automated adjustable lens may be adjusted to generate a sharp image of targets 48 on the optical sensor and subsequently adjust said tunable lens 64 or other motorless automated adjustable lens to set the objective plane a defined distance distal from front end optical aperture 63 such that a sample volume is located at said distance distal from front end optical aperture 63 is sharply imaged on the optical sensor. Thus, an autofocus operation may be realized.

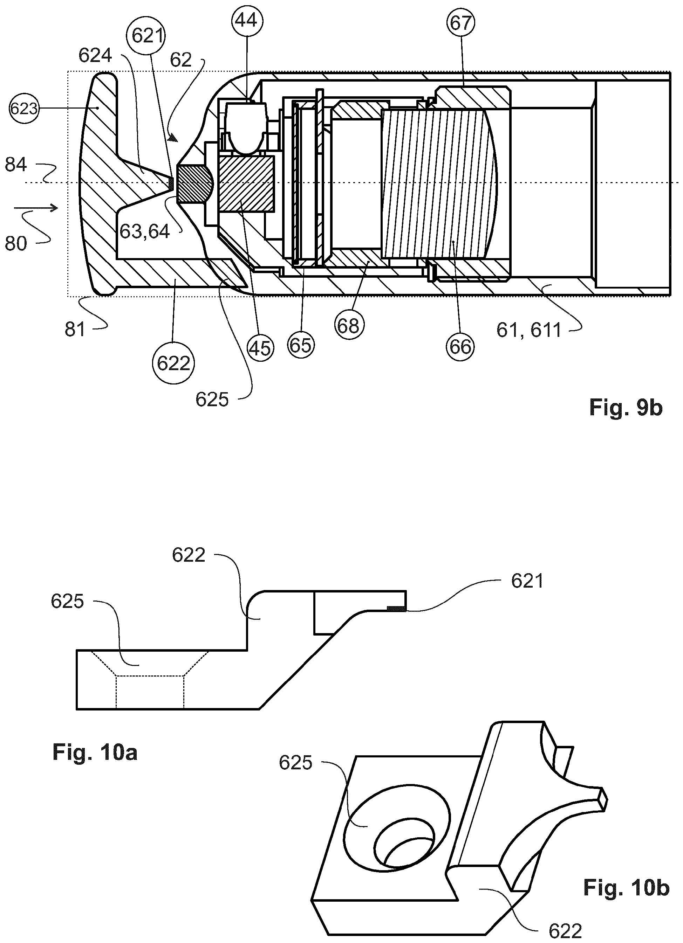

Figure 9a and 9b disclose a first and a second example of an objective lens unit comprising a reflector 621 and a bubble shield 623. The enclosure 61 of the objective lens unit forms the body of the device for directing illumination. An optical aperture 63 is arranged at the distal front end 62 of the body in the form of an enclosure 61. A solid immersion lens 64 is in the depicted embodiment used to close the optical aperture 63. Inside of the enclosure 61, there is an objective lens system, consisting of two lenses 65 and 66, wherein the lens 65 can be a tuneable lens. The objective lens system is configured to collect the light received through the optical aperture 63. The reflector 621 is arranged on the side of the bubble shield 623 facing the distal front end 62 of the body 61. The optical axis 84 of the objective lens system 65,66 and optical aperture 63 and the reflector 621 coincide.

The illustrated arrangement of the bubble shield 623 is such that the intended flow direction 80 of the streaming liquid in which the shown objective lens unit is supposed to be used in parallel to the optical axis 84. The bubble shield 623 is in the shown example a blunt body. It has a positive curvature on the side facing the flow direction 80 and is rotationally symmetric with the optical axis being the axis of symmetry. In the intended operating conditions, the optical aperture 63 is in the wake of the bubble shield 623. The effective cross sections are the cross sections perpendicular to the flow direction 80. The cross-section and the effective cross-section of the optical aperture 63 is smaller than the cross-section resp. the effective cross-section of the bubble shield 623. A circumferential outer wall of the objective lens unit is circular cylindrical. As indicated with a dashed line, the distal part of the objective lens unit, including the bubble shield 623 and the reflector 621, fits in the volume of a circular cylinder 81 with an outer diameter of, in this case, 12 mm. The bubble shield 623 is connected to the body 61 with a strut 622. The circumferential extent of the strut 622, measured around the flow direction 80 respectively the symmetry axis of the circular cylinder 81 as axis, is about 10° ensuring thereby on the one hand sufficient mechanical stability and on the other hand minimizing the effect on the liquid flow. Strut 622, bubble shield 623 and the reflector 621 form a reflector assembly.

The body 61 has in the embodiments of figures 9a and 9b and along the optical axis 84, in the direction from distal to proximal, a steadily increasing cross-section: It has its minimal value at the optical aperture 63. The region of the optical aperture 63 forms a first plateau which increases steadily in the distal -proximal direction. Starting from the maximal cross section, here being the cross section of the circular cylinder 81, the cross section reduces steadily in the proximal-distal direction. The increase and the decrease are matched such that a smooth and steady transition is archived. In the depicted example the regions of the body with increase and the decrease of the cross-section have a positive curvature while the region where the transition occurs has a negative curvature. Such a shape exposes the optical aperture 63 while still providing sufficient volume inside of the body or enclosure 61 in the vicinity to it, allowing to arrange the objective lens system close to the optical aperture 63.

In the embodiment shown in Figure 9a, the body 61 comprises an additional aperture 82 through which light from the illumination source 44 is emitted onto the reflector 621. The reflector 621 is a reflective coating applied onto the proximal side of the bubble shield 623. The illumination source 44 is an LED arranged in the body 61 or enclosure in the shown example. In other embodiments is could also be arranged at a different location and the light is guided by light guides such as fiber optics to the additional aperture 82. The additional aperture 82 is closed with a window or a lens. In other embodiments, the additional aperture 82 cloud also be formed directly be the illumination source 44 or suitable light guides.

In the embodiment shown in Figure 9b, the illumination is similar to the one discussed with respect to Figure 5, the objective lens unit comprising an illumination source 44 and beam splitter 45 as a means for coupling light from illumination source 44 into objective lens unit 6. Beam splitter 45 is arranged in the optical path of objective lens unit 6, while illumination source 44 is arranged lateral of the optical path. As will be appreciated, beam splitter 45 deflects part of the light from illumination source 44 in a forward or distal direction of objective lens unit 6 and towards immersion lens 64. The bubble shield 623 of this embodiment further comprises a flow divider 624. The reflector 621, for example a cat eye retroreflector, is arranged in the tip of the flow divider 624. The bubble shield 623 with the flow divider 624 is rotationally symmetric with the optical axis being the axis of symmetry. The flow divider 624 has a conical shape, with its radial extend decreasing along the distal-proximal direction. Figure 9b depict further an example of a connecting means 325 of the reflector: the strut 622 is received in a recess in the body 61 locked therein.

Figures 10a and 10b illustrate another example of a reflector assembly: It comprises a cuboid basis in which the connecting means 625 are arranged. The cuboid basis has a thickness perpendicular to a basis surface. The top surface is arranged parallel to the basis surface in a distance equal to the thickness. In the depicted example, the connecting means 625 it is a recess to receive a screw. The recess extends in the thickness direction of the basis. The diameter of the recess is greater in the top surface than in the basis surface. The strut 622 extends from one side of this basis in the thickness direction. It has a height measured from the basis surface and in the direction of the thickness of about twice the thickness of said basis. The strut 622 comprises a lower part, which has the shape of a triangular prism, with one side being equal to the side of eh cuboid basis form where it starts, and an upper part which resembles a second cuboid from which two circular cylinder segments are cut-away so that a cantilever structure is created. The cuboid basis and the second cuboid are arranged parallel to each other but shifted so that the section comprising the cut-aways is not over the basis while the section without the cut-aways contact a second side of the triangular prism. The thickness of the second cuboid is about the same as the thickness of the cuboid basis. The reflector 621 is embedded in the tip of the cantilever formed by the cut-aways. It is arranged on the lower side, reflecting light in the thickness direction downwards, i.e. in the direction from the top surface to the basis surface. Such a reflector assembly can efficiently be produced, can easily be mounted to the body and provides sufficient stability. To provide a smooth surface in contact with the measurement medium, the microscope or the objective lens unit or the assembly in which this reflector is used can comprise a cover to cover connecting means and similar small-scale structures.

While the subject matter of the disclosure has been explained by means of exemplary embodiments, it is understood that these are in no way intended to limit the scope of the claimed invention. It will be appreciated that the claims cover embodiments not explicitly shown or disclosed herein, and embodiments deviating from those disclosed in the exemplary modes of carrying out the teaching of the present disclosure will still be covered by the claims.

**Reference signs list**

| | |
|---|---|
| 1 | Microscope |
| 2 | Tank, bioreactor |
| 3 | Sample liquid |
| 4 | Flange |
| 5 | Head of microscope |
| 6 | Objective lens unit |
| 7 | Shaft |
| 11 | Distal end of microscope |
| 21 | Wall of tank or bioreactor |
| 44 | Illumination source |
| 45 | Means for coupling light from illumination source into objective lens unit; beam splitter |
| 48 | Target |
| 51 | Camera |
| 52 | Optical sensor |
| 53 | Illumination source |
| 54 | Means for coupling light from illumination source into objective lens unit; beam splitter |
| 61 | Enclosure |
| 62 | Distal front end of objective lens unit |
| 63 | Front end optical aperture of objective lens unit |
| 64 | Solid immersion lens |
| 65 | Optical component, motorless automated adjustable lens |
| 66 | Optical component, aspheric lens |
| 67 | Threaded sleeve |
| 68 | Distance sleeve |
| 69 | Air gap |
| 71 | O-ring sealing |
| 72 | O-ring sealing |
| 611 | Cap |
| 612 | Sleeve |
| 613 | Flange of cap |
| 621 | Reflector |
| 622 | Strut |
| 623 | Bubble Shield |
| 624 | Flow divider |
| 625 | Connecting means of reflector |
| 641 | Plan window |
| 642 | Immersion lens |
| 643 | Liquid-filled gap |
| 80 | Flow direction |
| 81 | Cylinder |
| 82 | Additional aperture |
| 84 | Optical axis |
| 100 | Sample volume |
| s | Distance from front end of objective lens unit to sample volume |

## Claims

1. Device for directing illumination, the device comprising a body (61, 611), the body having a distal front end (62) and an optical aperture provided (63) in the front end, and a reflector (621) connected to the body and arranged distal from the front end and a non-zero distance from the optical aperture and is configured to receive light emitted through the front end and to reflect at least part of the light received from the front end back into the optical aperture.

2. Device according to any preceding claim, wherein the reflector is connected to the body by at least one strut (622).

3. Device according to any preceding claim suitable to be arranged in a streaming liquid to be measured, whereby the device is equipped with a bubble shield, whereby the bubble shield has a cross section greater than the one of the optical aperture and is arranged distal of the distal front end of the body and whereby the reflector is preferably part of the bubble shield.

4. Device according to claim 3, whereby the bubble shield has a positive curvature on the side facing the flow direction or the side facing away from the optical aperture.

5. Device according to any one of claims 3 to 4, whereby the bubble shield comprises a flow divider, which extends towards the optical aperture, and whereby the reflector is preferably mounted to said flow divider.

6. An assembly comprising
a. an objective lens unit (6) and
b. a device for directing illumination according to any of the preceding claims,
c. the objective lens unit comprising
i. an enclosure, the enclosure having a distal front end,
ii. a front end optical aperture (63) of the objective lens unit provided in the distal front end (62) of the objective lens unit,
iii. the objective lens unit further comprising an objective lens system (65, 66) arranged inside the enclosure (61) and proximal from the front end optical aperture (63) of the objective lens unit,
iv. wherein the objective lens system is configured to collect light received through the front end optical aperture (63) of the objective lens unit,
d. wherein the device for directing illumination is attached to the objective lens unit,
i. wherein the device for directing illumination is arranged such that the reflector (621) is arranged distal from the front end (62) of the objective lens unit and a non-zero distance from the front end optical aperture (63) of the objective lens unit and is arranged to reflect at least part of the light received from the front end of the body back into the optical aperture of the objective lens unit
e. whereby device for directing illumination and/or the objective lens unit or the combination of both is preferably liquid-proof, such that the assembly is suitable to be used when at least partially submerged in a liquid.

7. An objective lens unit (6), the objective lens unit comprising
a. an enclosure (61),
i. wherein at least a part of the enclosure (61) is formed by the body (611) of a device for directing illumination according to any of claims 1 through 5,
ii. wherein the front end of the body of the device for directing illumination is a distal front end (62) of the enclosure and
iii. the optical aperture of the body of the device for directing illumination is a front end optical aperture (63) of the objective lens unit, and
iv. whereby the reflector (621) of the device for directing illumination is connected to the enclosure (61), and
b. the objective lens unit further comprising an objective lens system (65, 66) arranged inside the enclosure (61) and proximal from the front end optical aperture (63) of the objective lens unit and wherein the objective lens system is configured to collect light received through the front end optical aperture (63)
c. whereby the objective lens unit is preferably suitable to be used when at least partially submerged in a liquid.

8. Objective lens unit according to the preceding claim, the enclosure (61) comprising a cap (611) and a sleeve (612), wherein the objective lens system (65, 66) is provided inside the sleeve, wherein the cap (611) is provided by the body of the device for directing illumination, the cap (611) comprising a lateral sheath, a front wall and a rear port, wherein the front end optical aperture (63) of the objective lens unit is provided in the front wall of the cap (611), wherein the sleeve (612) is preferably at least partially received inside the cap (611).

9. Device, assembly or objective lens unit according to any of the preceding claims, wherein the objective lens unit (6) or the body (6) comprises a proximal rear connector interface, wherein at least a section of the objective lens unit (6) or the body (6) excluding the rear connector interface is liquid-proof.

10. Device, assembly or objective lens unit according to any of the preceding claims, comprising a sample illumination unit, wherein the sample illumination unit comprises
a. an illumination source (44) and
b. a means (45) for coupling light from the illumination source (44) into the objective lens unit (6) and/or into the body,
c. wherein in particular the means for coupling light from the illumination source into the objective lens unit is arranged distal from the objective lens system (65, 66) and proximal from the front end optical aperture (63) of the objective lens unit and is further configured to project said light into a proximal-distal direction of the objective lens unit through the front end optical aperture and onto the reflector (621).

11. A reflector assembly (621), comprising a reflector and at least one strut, whereby the reflector is attached to at least one strut, and the at least one strut is configured to be mounted to the enclosure of an objective lens unit according to any one of claims 7 or 8, wherein the reflector assembly is sized and shaped such that the reflector is positioned in a non-zero distance distal from a front end of the enclosure of the objective lens unit and is positioned and configured to receive light emitted through the front end of the objective lens unit and to reflect at least part of the light received back into the optical aperture when the reflector assembly is attached to the enclosure of said objective lens unit.

12. Microscope (1) for in-situ application, the microscope comprising an assembly or an objective lens unit (6) according to any of claims 6 through 10 and an optical sensor (52), wherein the optical sensor is functionally coupled to the objective lens unit to receive light transmitted from a front side of the objective lens unit and through the objective lens unit.

13. Microscope (1) for in-situ application according to the preceding claim, wherein the microscope (1) comprises a sample illumination unit comprising an illumination source (44) and a means (45) for coupling light from the illumination source (44) into the objective lens unit (6), wherein said means for coupling light from the illumination source into the objective lens unit is preferably arranged proximal from the objective lens unit.

14. Method of performing cytometry using a microscope according to any of claims 12 or 13, the method comprising
submerging at least a distalmost front section of the objective lens unit (6) including the distal front end (21) and the front end optical aperture (63) of the objective lens unit or the distal front end (62) of the body including the optical aperture (63) and the reflector in a sample liquid (3), preferably contained inside the bioreactor,
emitting light through the front end of the objective lens unit or through the front end of the body, said emitted light directed in a direction towards the reflector (621),
reflecting at least a part of the light that hits the reflector, back to the front end optical aperture (63) of the objective lens unit and/or the optical aperture of the body and through a sample volume (100) of the sample liquid, and
recording at least one image generated by the objective lens unit (6) using the optical sensor (52),
wherein preferably the at least one image includes forward scattered light and backward scattered light from objects contained in the sample volume.

15. Method according to the preceding claim, the method comprising setting a focal length of the objective lens unit such that the reflector is imaged on the optical sensor and issuing a warning, if the observed reflectivity is lower than an expected value and/or if the image shows unexpected structures.
